# EUROPÄISCHE PATENTANMELDUNG

(11) **EP 1 916 005 A1**
(43) Veröffentlichungstag der Anmeldung: **30.04.2008**
(21) Anmeldenummer: 07118559.9
(22) Anmeldetag: 16.10.2007
(51) Int. Cl.: A61L 9/03, A61L 9/012

(54) **Duftemitter**

(30) Priorität: 16.10.2006 DE 102006048758
(71) Anmelder: Ruetz, Stefan, 80807 München (DE)
(72) Erfinder: Horn Dr., Andreas, 85737, Ismaning (DE)
(74) Vertreter: Zipse Habersack Kritzenberger

(57) **Zusammenfassung**

Die Erfindung betrifft eine Vorrichtung und ein Verfahren zum Austragen eines Duftstoffes, bei der die Duftausgabe dadurch erfolgt, dass ein begrenztes Luftvolumen hinter einem Duftspeicher schlagartig erwärmt wird, wobei die sich ausdehnende Luft den Duftspeicher passiert und durch eine feine Blende oder Kapillare nachfolgend entweichen kann und dabei den Duftstoff an die Umgebung abgibt.

## Beschreibung

### I.

Duftgeräte verwenden in aller Regel einen Ventilator zum Transport der bedufteten Luft aus dem Duftemitter an die Umgebung. Diese Lüfter sind teuer und erzeugen Geräusche, die in stiller Umgebung störend wirken können.

Mit der Erfindung wird der Duft unter Verzicht eines Lüfters ausgebracht, die Duftabgabe bleibt dabei aber weiterhin steuerbar.
Erfindungsgemäß wird die Duftausgabe dadurch bewerkstelligt, dass ein begrenztes Luftvolumen hinter einem Duftspeicher (z. B. Duftgranulat) schlagartig erwärmt wird (siehe Abbildung). Die sich ausdehnende Luft kann durch eine feine Blende oder Kapillare entweichen und transportiert dabei den Duftstoff an die Umgebung. Die Anordnung von Heizung, Duftspeicher und Blende ist dabei so gewählt, dass die sich ausdehnende Luft den Duftspeicher auf dem Weg zur Blende durchströmen muß und so den Duft gut aufnehmen kann. Durch eine geeignete Dimensionierung von Kammervolumen, Blendenquerschnitt bzw. Kapillargeometrie sowie des Duftspeichermediums kann eine Duftabgabe von mehreren Sekunden erreicht werden. Nach der Duftausgabe kühlt sich die Heizung und die Luft im Kammervolumen ab und zieht sich dadurch zusammen, so dass in der Kammer ein Unterdruck entsteht, durch den frische Luft über die Blende / Kapillare in die Duftkammer eingesaugt wird. Der Betrieb des Duftgeräts ist also intermittierend vorgesehen, beispielsweise 8 Sekunden Duftausgabe / 22 Sekunden Abkühlphase. Aufgrund der stark unterschiedlichen thermischen Massen der erwärmten Luft (geringe Wärmekapazität) und des Duftspeichermediums (granularer Feststoff oder Duftgel mit vergleichsweise hoher Wärmekapazität) erwärmt sich der Duftstoff bei der Duftausgabe nur sehr wenig.

### II.

Im wesentlichen wird gegenüber dem Stand der Technik mit Lüfter bei der Erfindung der Lüfter durch eine einfache Heizspirale ersetzt. Der Bauraum verändert sich dadurch nur unwesentlich. Die Kosten für die Heizspirale sind geringer, Geräusche entstehen bei der Duftabgabe nicht. Die Steuerung erfolgt (wie auch beim Lüfter) durch einen Port eines Mikrokontrollers mit Leistungsausgang.

### Bezugszeichen

- 1: Abdichtung
- 2: Glühwendel
- 3: Kapillare
- 4: Kolben (Spritze)

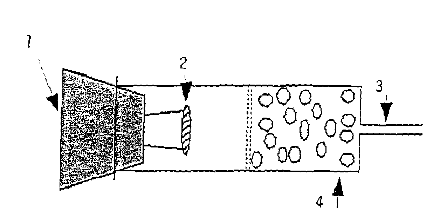

## Patentansprüche

1. Duftemitter zum Austragen eines Dufts aufweisend eine Luft-Heizung für ein begrenztes Luftvolumen, einen Duftspeicher, und eine Blende oder Kapillare, welche Bauteile in Reihe angeordnet sind, derart, dass das mittels der Heizung erwärmbare begrenzte Luftvolumen den Duftspeicher bei Wärmeausdehnung der Luft durchströmt und an der Blende bzw. Kapillare austritt.

2. Duftemitter nach Anspruch 1,
**dadurch gekennzeichnet, dass** eine Heizkammer zur Aufnahme des begrenzten Luftvolumens vorgesehen ist, welche Kammer im Betrieb des Duftemitters mit der Außenumgebung über die Blende/Kapillare in Verbindung steht.

3. Duftemitter nach einem der Ansprüche 1 oder 2,
**dadurch gekennzeichnet, dass** der Duftspeicher mittels einer luftdurchlässigen Wand von der Heizung bzw. der Heizkammer getrennt ist.

4. Duftemitter nach einem der vorangehenden Ansprüche,
**dadurch gekennzeichnet, dass** die Duftabgabe durch einen Port eines Mikrokontrollers mit Leistungsausgang steuerbar ist.

5. Verfahren zum Ausbringen eines Duftes, bei dem ein begrenztes Luftvolumen hinter einem Duftspeicher schlagartig erwärmt wird und die sich ausdehnende Luft den Duftspeicher durchströmt, wobei die Luft durch eine Blende oder Kapillare entweicht und dabei den Duftstoff an die Umgebung abgibt.

6. Verfahren nach Anspruch 5,
**dadurch gekennzeichnet, dass** sich die Luft in einem Kammervolumen und die Heizung nach der Duftausgabe abkühlen, und **dadurch** in der Kammer ein Unterdruck entsteht, durch den frische Luft über die Blende/Kapillare in die Duftkammer eingesaugt wird.

7. Verfahren nach einem der Ansprüche 5 oder 6,
**dadurch gekennzeichnet, dass** der Betrieb intermittierend erfolgt.

8. Verfahren nach einem der Ansprüche 5 bis 7,
**dadurch gekennzeichnet, dass** ein Duftspeichermedium mit gegenüber der Luft vergleichsweise hoher spezifischer Wärmekapazität verwendet wird.
